# EUROPEAN PATENT APPLICATION

(11) **EP 3 437 695 A1**
(43) Date of publication of application: **06.02.2019**
(21) Application number: 16897196.8
(22) Date of filing: 28.10.2016
(51) Int. Cl.: A61N 5/06, A61H 23/02

(54) **WEARABLE DEVICE FOR REDUCING BODY FAT USING LEDS AND METHOD FOR OPERATING SAME**

(30) Priority: 29.03.2016 KR 20160037593; 18.04.2016 KR 20160047139
(71) Applicant: Double H Ltd., Gyeonggi-do 13558 (KR)
(72) Inventor: LEE, Kyung Han, Yongin-si Gyeonggi-do 16909 (KR)
(74) Representative: Gevers Patents
(86) International application number: PCT/KR2016/012217
(87) International publication number: WO 2017/171175

(57) **Abstract**

Disclosed is a wearable device capable of reducing a body fat. The wearable device includes a first light emitting diode (LED) source configured to emit a first light having first wavelengths; a second LED source configured to emit a second light having second wavelengths; vibration motors; a microprocessor configured to interpret an operation control signal and to generate a first control signal and a second control signal based on a result of the interpreting; and a battery configured to supply operating voltages to the first LED source, the second LED source, the vibration motors, and the microprocessor. At least one of the first LED source and the second LED source is turned on in response to the first control signal, at least one of the vibration motors is turned on in response to the second control signal, and the microprocessor is configured to accumulate and record a first on-time of the first LED source, a second on-time of the second LED source, and third on-times of the vibration motors.

## Description

### TECHNICAL FIELD

Embodiments relate to a wearable device that may reduce a body fat, and more particularly, to a wearable device that may reduce a body fat and measure the body fat using light emitting diodes (LEDs) and a method of controlling the wearable device using a mobile device.

### RELATED ART

Unlike the obesity by subcutaneous fat, abdominal obesity indicates that the fat is excessively accumulated in the abdomen's internal organs. Since the fat in the abdomen of the body is close to the liver compared to the fat distributed elsewhere in the body, the abdominal obesity may highly affect a human body.

The main cause of abdominal obesity is the ingestion of excessive food, for example, calories. In particular, the excessive intake of fat or fat components may be the main cause of abdominal obesity. When a person over-consumes carbohydrate, the carbohydrate is converted to the fat and the carbohydrate also causes the abdominal obesity.

The lack of exercise may cause the abdominal obesity. The lack of exercise may cause a muscle weakness and a fat accumulation in the abdomen. If the muscle mass increases by an amount of exercise, the basic metabolism of the human body may increase and the fat may decrease. However, it may be difficult to do exercise steadily.

Although a vibration belt is proposed to reduce the abdominal obesity, the effect of reducing the abdominal obesity using the vibration belt is small since a user is still and consumes little calories and only the vibration belt is vibrating

### DESCRIPTION

### OBJECTS

An aspect of at least one example embodiment provides a wearable device that may effectively reduce a body fat causing the abdominal obesity of a user using a light that is output from light emitting diodes (LEDs) and may provide the effect of the aerobic exercise to the user using vibration motors, and a method of controlling the wearable device using a mobile device.

### SOLUTIONS

According to an aspect of at least one example embodiment, there is provided a wearable device including a first light emitting diode (LED) source configured to emit a first light having first wavelengths; a second LED source configured to emit a second light having second wavelengths; vibration motors; a microprocessor configured to interpret an operation control signal and to generate a first control signal and a second control signal based on a result of the interpreting; and a battery configured to supply operating voltages to the first LED source, the second LED source, the vibration motors, and the microprocessor. At least one of the first LED source and the second LED source is turned on in response to the first control signal, at least one of the vibration motors is turned on in response to the second control signal, and the microprocessor is configured to accumulate and record a first on-time of the first LED source, a second on-time of the second LED source, and third on-times of the vibration motors.

According to an aspect of at least one example embodiment, there is provided a wearable device in a structure to be wearable around the waist of a user to measure a body fat of the user, the wearable device including a first LED source configured to emit a first light having first wavelengths toward the waist; a second LED source configured to emit a second light having second wavelengths toward the waist; vibration motors configured to provide a vibration to the waist; an input device configured to provide a user interface to the user; a wireless transceiver configured to wirelessly communicate with a mobile device; a body fat sensors configured to measure the body fat; and a microprocessor configured to interpret an operation control signal input through one of the input device and the wireless transceiver and to generate a first control signal and a second control signal based on a result of the interpreting. At least one of the first LED source and the second LED source is turned on in response to the first control signal, at least one of the vibration motors is turned on in response to the second control signal, and the microprocessor is configured to accumulate and store a first on-time of the first LED source, a second on-time of the second LED source, and third on-times of the vibration motors.

According to an aspect of at least one example embodiment, there is provided a method of controlling an operation of a wearable device using the wearable device in a structure to be wearable around the waist of a user to measure a body fat of the user and a mobile device capable of wirelessly communicating with the wearable device, the method including receiving, by a microprocessor included in the wearable device, an operation control signal input through one of an input device included in the wearable device and a wireless transceiver included in the wearable device; selecting, by the microprocessor, one of operation modes of the wearable device from a memory device configured to store information associated with the operation modes using the operation control signal, and to generate a first control signal and a second control signal based on the selected operation mode; turning on at least one of a first light emitting diode (LED) source and a second LED source included in the wearable device in response to the first control signal, and turning on at least one of vibration motors included in the wearable device in response to the second control signal; accumulating and recording, by the microprocessor, a first on-time of the first LED source, a second on-time of the second LED source, and third on-times of the vibration motors; receiving, by the microprocessor, a state request signal transmitted from the wireless communication device through the wireless transceiver; and transmitting, by the microprocessor, at least one of an operation time of the wearable device, the accumulated first on-time, the accumulated second on-time, and the accumulated third on-times to the mobile device through the wireless transceiver in response to the state request signal.

### EFFECTS

A wearable device according to example embodiments may reduce a body fat and may measure the body fat using light emitting diodes (LEDs) included in the wearable device.

A wearable device according to example embodiments may decompose triglyceride into fatty acid and glycerol by irradiating an LED light toward the abdomen of a user and at the same time, may quickly burn the decomposed fatty acid and glycerol by vibrating the abdomen using vibration motors. Accordingly, the wearable device may directly reduce the abdominal fat. That is, although the user does not do aerobic exercise, the effect as if the user is doing the aerobic exercise, that is, the diet effect may be maximized.

According to example embodiments, a user may control the wearable device using a mobile device, the convenience of using the wearable device may be enhanced.

### DETAILED DESCRIPTION OF DRAWINGS

The detailed description of drawings is provided to further sufficiently understand the drawings used in the detailed description.
FIG. 1 is a perspective view of a wearable device according to an example embodiment.
FIG. 2 is a top view of the wearable device of FIG. 1.
FIG. 3 illustrates a system that includes the wearable device of FIG. 1 and a mobile device for controlling the wearable device.
FIG. 4 is a block diagram of an electronic system configured in the wearable device of FIGS. 1 and 2.
FIG. 5 illustrates an example of information representing operation modes stored in a memory device of FIG. 4.
FIG. 6 illustrates timing diagrams of control signals of FIG. 4.
FIG. 7 is a table showing an example of on-times accumulated in a microprocessor of FIG. 4.
FIG. 8 illustrates an example of a graphic user interface (GUI) of an application executed on the mobile device of FIG. 3.
FIG. 9 illustrates an example of a GUI showing a change in a body fat provided from an application executed on the mobile device of FIG. 3.
FIG. 10 is a flowchart illustrating an operation of the wearable device of FIG. 4.
FIG. 11 is a flowchart illustrating a process of acquiring state information from the wearable device using an application executed on the mobile device of FIG. 3.
FIG. 12 is a flowchart illustrating a process of controlling the wearable device using an application executed on the mobile device of FIG. 3.
FIG. 13 is a flowchart illustrating a process of controlling the wearable device using an application executed on the mobile device of FIG. 3.
FIG. 14 illustrates a wearable device according to an example embodiment worn around a waist portion of a user.
FIG. 15 illustrates a wearable device according to an example embodiment worn around a waist portion of a user.
FIG. 16 is a block diagram illustrating an example of an electronic device included in the wearable device of FIGS. 14 and 15.
FIG. 17 illustrates an operation of a controller of FIG. 16.
FIG. 18 is a block diagram illustrating another example embodiment of an electronic device included in the wearable device of FIGS. 14 and 15.
FIG. 19 illustrates an operation of a controller of FIG. 18.

### MODE

The following structural or functional description of example embodiments is provided to describe the example embodiments and the example embodiments may be implemented in various forms and are not construed as being limited to the disclosure.

Various alterations and modifications may be made to the example embodiments. Accordingly, the example embodiments are illustrated in the drawings and described in detail herein. However, the example embodiments are not construed as being limited to specific example embodiments and should be understood to include all changes, equivalents, and replacements within the technical scope of the disclosure.

Terms, such as first, second, and the like, may be used herein to describe components. Each of these terminologies is not used to define an essence, order or sequence of a corresponding component but used merely to distinguish the corresponding component from other component(s). For example, without departing from the scope of the disclosure, a first component may be referred to as a second component, and similarly the second component may also be referred to as the first component.

It should be noted that if it is described that one component is "connected", "coupled", or "joined" to another component, a third component may be "connected", "coupled", and "joined" between the first and second components, although the first component may be directly connected, coupled, or joined to the second component. On the contrary, it should be noted that if it is described that one component is "directly connected", "directly coupled", or "directly joined" to another component, a third component may be absent. Expressions describing a relationship between components, for example, "between", directly between", or "directly neighboring", etc., should be interpreted to be alike.

The terminologies used herein are used to simply describe specific example embodiments and are not construed to limit the disclosure. The singular forms "a", "an", and "the" are intended to include the plural forms as well, unless the context clearly indicates otherwise. It will be further understood that the terms "comprises/comprising" and/or "includes/including" when used herein, specify the presence of stated features, integers, steps, operations, elements, and/or components, but do not preclude the presence or addition of one or more other features, integers, steps, operations, elements, components and/or groups thereof.

Unless otherwise defined, all terms, including technical and scientific terms, used herein have the same meaning as commonly understood by one of ordinary skill in the art to which this disclosure pertains. Terms, such as those defined in commonly used dictionaries, are to be interpreted as having a meaning that is consistent with their meaning in the context of the relevant art, and are not to be interpreted in an idealized or overly formal sense unless expressly so defined herein.

Hereinafter, example embodiments are described with reference to the accompanying drawings.

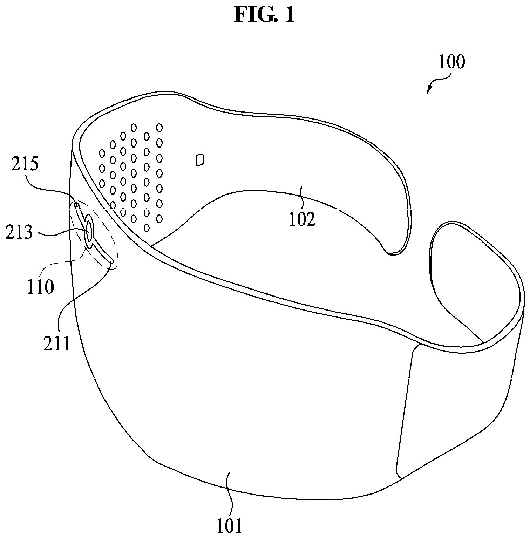

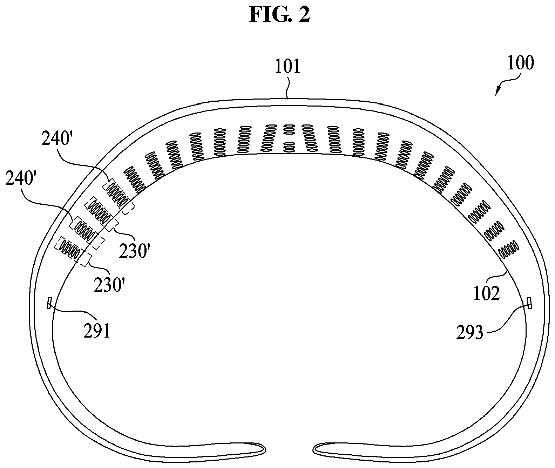

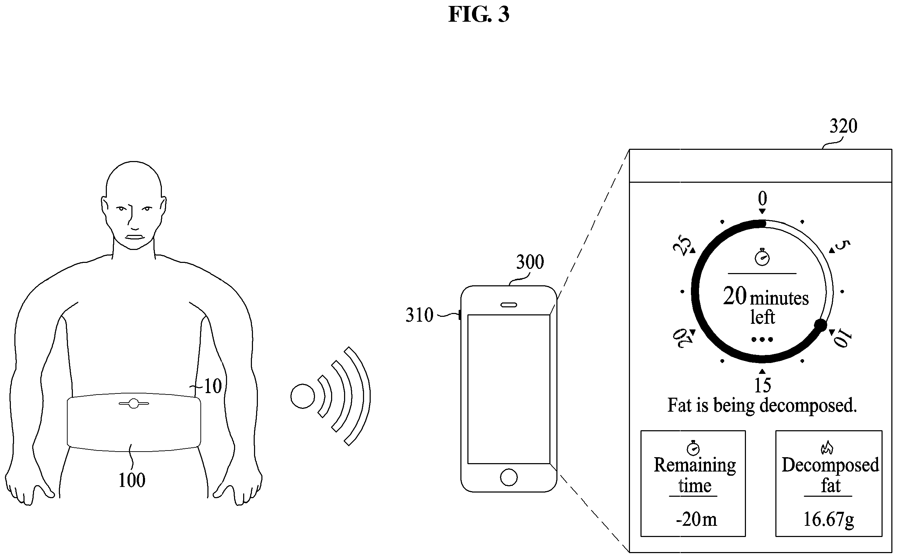

FIG. 1 is a perspective view of a wearable device according to an example embodiment, FIG. 2 is a top view of the wearable device of FIG. 2, and FIG. 3 illustrates a system that includes the wearable device of FIG. 1 and a mobile device for controlling the wearable device.

Referring to FIGS. 1 through 3, a wearable device 100 herein refers to a device being worn around a waist or a waist portion of a user 10 and capable of decomposing a body fat, particularly, an abdominal fat of the user 10 and may indicate, for example, a diet belt or a wearable light therapy device. Light therapy refers to a technique applicable to health, beauty, anti-aging, skin care, reduction in obesity, and/or disease prevention using wavelengths corresponding to a specific color of visible light.

A body of the wearable device 100 may be formed using an elastic material. A user interface 110 capable of controlling an operation of the wearable device 110 may be provided on a front surface 101 of the wearable device 100, and the user interface 110 may include a plurality of buttons 211, 213, and 215.

For example, the user 10 wearing the wearable device 100 around the waist of the user 10 may change power-on, power-off, and an operation mode by manipulating or pressing the respective buttons 211, 213, and 215. The button 213 is a button capable of controlling or manipulating power-on and power-off of the wearable device 100. Although FIG. 1 illustrates three buttons 211, 213, and 215, they are provided for clarity of description only. A number of buttons associated with the operation of the wearable device 100 and locations of the buttons may be variously changed based on design specifications.

A mobile device 300 may perform wireless communication with the wearable device 100 and may control an operation of the wearable device 100 through the wireless communication. The mobile device 300 may be configured as a smartphone, a tablet personal computer (PC), a wearable computer, a mobile Internet device (MID), an Internet of Things (IoT) device, or an Internet of Everything (IoE) device.

The mobile device 300 may execute an application program (or an application (app)) 320 capable of controlling the operation of the wearable device 100. The mobile device 300 and the wearable device 100 may be paired through a pairing process.

The user 10 of the wearable device 100 may request the wearable device 100 for state information of the wearable device 100 using the application 320 displayed on a display 310 of the mobile device 300, or may measure a body fat, particularly, a body fat around an abdominal portion of the user 10 or may control an operation of each of light emitting diode (LED) sources and vibration motors provided to the wearable device 100 using the wearable device 100.

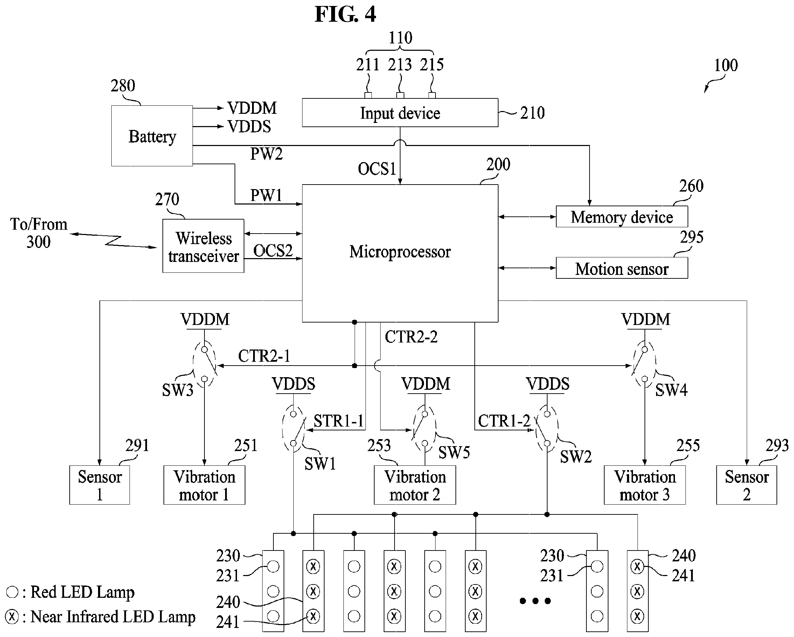

FIG. 4 is a block diagram of an electronic system included in the wearable device of FIGS. 1 and 2. Referring to FIGS. 1 through 4, an electronic system configured in the wearable device 100 may include a microprocessor 200, the user interface 110 including the buttons 211, 213, and 215, an input device 210, first LED sources 230, second LED sources 240, vibration motors 251, 253, and 255, a memory device 260, a wireless transceiver 270, a battery 280, sensors 291 and 293, a motion sensor 295, and switches SW1, SW2, SW3, SW4, and SW5. Although FIG. 4 illustrates that the electronic system includes the motion sensor 295, the motion sensor 295 may not be provided depending on example embodiments.

The microprocessor 200 may refer to a controller, a processor, or a central processing unit (CPU) capable of controlling the overall operation of the wearable device 100 or the electronic device 110. An operation of the microprocessor 200 is described in association with an operation of each of the components 210, 230, 240, 251, 253, 255, 260, 270, 280, 291, and 293.

The microprocessor 200 may receive and interpret an operation control signal OCS1 or OCS2 output from the input device 210 or the wireless transceiver 270, and may generate a first control signal and a second control signal based on a result of the interpreting. The wireless transceiver 270 may be a transceiver using a Bluetooth communication technology, however, is not limited thereto.

The user 10 wearing the wearable device 100 around the waist may change power-on, power-off, and/or an operation mode of the wearable device 100 using the respective buttons 211, 213, and 215 included in the user interface 110.

The input device 210 may transmit, to the microprocessor 200, the operation control signal OCS1 that is generated in response to manipulating or pressing each of the buttons 211, 213, and 215. The input device 210 may function as a receiver.

The first LED sources 230 may be provided on a rear surface 102 of the wearable device 100, that is, to face the waist or the abdomen of the user 10 wearing the wearable device 100, and may output a first light having first wavelengths toward the waist through holes 230' formed in the rear surface 102. The first LED sources 230 may include first LED lamps 231. For example, each of the first LED lamps 231 may be an LED lamp configured to generate an Red ray.

The second LED sources 240 may be provided on the rear surface 102 of the wearable device 100, that is, to face the waist or the abdomen of the user 10 wearing the wearable device 100, and may output a second light having second wavelengths toward the waist through holes 240' formed in the rear surface 102. The second LED sources 240 may include second LED lamps 241. For example, each of the second LED lamps 241 may be an LED lamp configured to generate a near infrared (NIR) ray. A portion of the first wavelengths and a portion of the second wavelengths may be the same. Each of the first LED sources 230 and each of the second LED sources 240 may be alternately provided at the same interval, however, are not limited thereto.

The Red ray and/or the NIR ray may penetrate into the skin around the waist of the user 10 and may be transferred to fat cells of panniculus.

Once a light, for example, the Red ray and/or NIR ray, output from the each LED lamp 231 and/or 241 is emitted toward the waist or the abdomen of the user 10, the light may stimulate mitochondria within fat cells and the mitochondria may generate energy for decomposing a fat. Such a chemical variation may promote the secretion of lipase enzymes that decompose triglyceride into fatty acid and glycerol. A lipid material decomposed into small pieces is discharged through pores formed on the surface of the fat cells, and the discharged lipid material may naturally decline or be combusted by a movement of the user 10 while circulating in a lymphatic system of the body.

If the wearable device 100 applies a vibration to the waist portion using at least one of the vibration motors 251, 253, and 255 while emitting a light toward the waist or the abdomen of the user 10 using the LED lamp 231 and/or 241, the mobility of fat cells may increase and the lipid material decomposed into small pieces may move smoothly in the body. Accordingly, the vibration may decrease the abdominal fat in replacement of aerobic exercise.

Referring to FIG. 4, the first LED lamps 231 and the second LED lamps 241 may be alternately provided. Depending on example embodiments, a center-to-center interval between the first LED lamp 231 and the second LED lamp 241 may be configured to be the same or may be configured to be different.

The first LED sources 230 may be turned on in response to a first source control signal CTR1-1. The second LED sources 240 may be turned on in response to a second source control signal CTR1-2. Here, the first control signal refers to all of the first and second source control signals CTR1-1 and CTR1-2. Accordingly, at least one of the first LED sources 230 and the second LED sources 240 may be turned on in response to the first control signal CTR1-1 and/or the second control signal CTR1-2.

For example, if the first source control signal CTR1-1 is activated, a first switch SW1 may supply a source operating voltage VDDS to the first LED sources 230 and each of the first LED sources 230 may emit a light using the source operating voltage VDDS. If the second source control signal CTR1-2 is activated, a second switch SW2 may supply the source operating voltage VDDS to the second LED sources 240 and each of the first LED sources 240 may emit a light using the source operating voltage VDDS.

The microcontroller 200 may receive and interpret the operation control signal OCS1 or OCS2 output from the input device 210 or the wireless transceiver 270, and may control an activation timing and an inactivation timing of each of the source control signals CTR1-1 and CTR1-2 based on an interpretation result.

The vibration motors 251 and 255 that perform functionality of micro vibrators may operate, for example, vibrate in response to an activated first motor control signal CTR2-1. The vibration motor 253 may operate, for example, vibrate in response to an activated second motor control signal CTR2-2. The second control signal refers to all of the motor control signals CTR2-1 and CTR2-2. Accordingly, at least one of the vibration motors 251 and 255 may be enabled or disabled in response to the second control signal (CTR2-1 and CTR2-2).

For example, if the first motor control signal CTR2-1 is activated, a third switch SW3 may supply a motor operating voltage VDDM to the first vibration motor 251 and the first vibration motor 251 may be enabled or vibrate. Also, if the first motor control signal CTR2-1 is activated, a fourth switch SW4 may supply the motor operating voltage VDDM to the third vibration motor 255 and the third vibration motor 255 may be enabled or vibrate. If the second motor control signal CTR2-2 is activated, a fifth switch SW5 may supply the motor operating voltage VDDM to the second vibration motor 253 and the second vibration motor 253 may be enabled or vibrate.

Although FIG. 4 illustrates an example in which two vibration motors 251 and 255 operate in response to the activated first motor control signal CTR2-1 and a single vibration motor 253 operates in response to the activated second motor control signal CTR2-2, a number of vibration motors provided in the wearable device 100 and an operation order of the vibration motors may vary based on example embodiments.

The microcontroller 200 may receive and interpret the operation control signal OCS1 or OCS2 output from the input device 210 or the wireless transceiver 270, and may control an activation timing and an inactivation timing of each of the motor control signals CTR2-1 and CTR2-2 based on an interpretation result.

The microcontroller 200 may execute firmware or software. The firmware or the software may perform a function of counting an operation time of the wearable device 100 when an operation of the wearable device 100 starts, a function of comparing a count value corresponding to a count result and a reference value, and a function of automatically turning off power supplied to the wearable device 100 when the count value is equal to the reference value. For example, the firmware or the software may control a power supply function of the battery 280.

While the power is being supplied to the microcontroller 200, the microcontroller 200 may accumulate and record a turn-on time (hereinafter, "first on-time") of the first LED sources 230, a turn-on time of the second LED sources 240 (hereinafter, "second on-time"), and/or turn-on times of the vibration motors 251, 253, and 255 (hereinafter, "third on-times). For example, firmware or software executed by the microcontroller 200 may accumulate and record the first on-time, the second on-time, and/or the third on-times.

The memory device 260 may store information associated with operation modes of the wearable device 100. The information may be stored in the memory device 260 in a table form. The memory device 260 may be configured as a nonvolatile memory device, such as a read only memory (ROM).

The microprocessor 200 may receive the operation control signal OCS1 or OCS2 output from the input device 210 or the wireless transceiver 270, and may analyze or use the operation control signal OCS1 or OCS2, may select one of operation modes MODE1 to MODEX stored in the memory device 260, and may generate the first control signal (CTR1-1 and CTR1-2) and the second control signal (CTR2-1 and CTR2-2) based on the selected operation mode. That is, the microprocessor 200 may control an activation timing and an inactivation timing of each of the control signals CTR1-1, CTR1-2, CTR2-1, and CTR2-2 based on the operation mode that is selected based on the operation control signal OCS1 or OCS2.

The wireless transceiver 270 may control or interface signals that are exchanged between the microprocessor 200 and the mobile device 300.

The microprocessor 200 may control power-on, power-off, and/or the operation mode of the wearable device 100 in response to signals output from the wireless transceiver 270. The signals may be signals corresponding to signals or commands that are output from the mobile device 300.

For example, when the mobile device 300 outputs a state request signal generated by the application 320, the microprocessor 200 may transmit, to the wearable device 300 through the wireless transceiver 270, state information that includes at least one of the first on-time, the second on-time, and the third on-times accumulated by the microprocessor 200 or the firmware executed by the microprocessor 200 in response to the state request signal transmitted from the wireless transceiver 270.

The battery 280 may output operating voltages PW1, PW2, VDDM, and VDDS. The operating voltage PW1 is supplied to the microprocessor 200, the operating voltage PW2 is supplied to the memory device 260, the motor operating voltage VDDM is supplied to the vibration motors 251, 253, and 255 through the respective corresponding switches SW3, SW4, and SW5, and the source operating voltage VDDS is supplied to the LED sources 230 and 240 through the respective corresponding switches SW1 and SW2. The battery 280 may further supply at least one operating voltage to at least one of the components 210, 270, 291, 293, and 295.

The body fat sensors 291 and 293 refer to sensors configured o measure a body fat, for example, a fat causing the abdominal obesity, of the user 10 that wears the wearable device 100 around the waist, and each of the body fat sensors 291 and 293 may include an electrode.

For example, when the mobile device 300 outputs a body fat measurement signal generated by the application 320, the microprocessor 200 may receive the body fat measurement signal through the wireless transceiver 270, may enable the body fat sensors 291 and 293 in response to the body fat measurement signal, may measure the body fat of the user 10 using measurement signals transmitted from the body fat sensors 291 and 293, and may transmit body fat data corresponding to a measurement result to the mobile device 300 through the wireless transceiver 270.

When the wearable device 100 further includes at least one motion sensor 295, at least one motion sensor 295 may measure a motion of the user 10 that wears the wearable device 100 around the waist.

For example, when the mobile device 300 outputs a motion measurement signal generated by the application 320, the microprocessor 200 may receive the motion measurement signal through the wireless transceiver 270, may enable the motion sensor 295 in response to the motion measurement signal, may measure the motion, for example, an operation and/or quantity of motion, of the user 10 using a measurement signal transmitted from the motion sensor 295, and may transmit motion data corresponding to a measurement result to the mobile device 300 through the wireless transceiver 270.

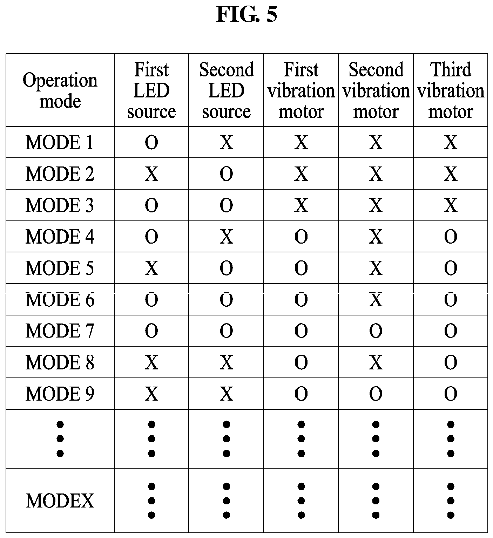

FIG. 5 illustrates an example of information indicating operation modes stored in a memory device of FIG. 4. Referring to FIGS. 1 through 5, the memory device 260 may store information associated with execution of each operation mode MODE1 to MODEX. Here, X denotes a natural number of 10 or more.

If the operation mode selected based on the operation control signal OCS1 or OCS2 is a first operation mode MODE1, the microprocessor 200 activates only the first source control signal CTR1-1 to turn on the first LED sources 230. If the operation mode selected based on the operation control signal OCS1 or OCS2 is a third operation mode MODE3, the microprocessor 200 activates all of the first source control signal CTR1-1 to turn on the first LED sources 230 and the second source control signal CTR1-2 to turn on the second LED sources 240.

If the operation mode selected based on the operation control signal OCS1 or OCS2 is a seventh operation mode MODE 7, the microprocessor 200 activates all of the control signals CTR1-1, CTR1-2, CTR2-1, and CTR2-2.

The first operation mode MODE1 is an operation mode configured to operate the first LED sources 230, the second operation mode MODE2 is an operation mode configured to operate the second LED sources 240, the third operation mode MODE3 is an operation mode configured to operate all of the first LED sources 230 and the second LED sources 240, the seventh operation mode MODE 7 is an operation mode configured to operate the first LED sources 230, the second LED sources 240, and the vibration motors 251, 253, and 255.

As described above, the microprocessor 200 or the firmware executed by the microprocessor 200 may search the memory device 260 for an operation mode corresponding to the operation control signal OCS1 or OCS2 that is received from the input device 210 or the wireless transceiver 270, and may control an activation timing and an inactivation timing of each of the control signals CTR1-1, CTR1-2, CTR2-1, and CTR2-2 corresponding to a search result.

For example, if a current operation mode is the third operation mode MODE3 and, in this instance, the user 10 presses the first button 211, the input device 210 transmits the operation control signal OCS1 instructing to press the first button 211 to the microprocessor 200. Accordingly, the microprocessor 200 or the firmware executed by the microprocessor 200 may control an activation timing and an inactivation timing of each of the control signals CTR1-1, CTR1-2, CTR2-1, and CTR2-2 to perform a fourth operation mode MODE4 in response to the operation control signal OCS1.

However, if a current operation mode is the third operation mode MODE3 and, in this instance, the user 10 presses the third button 215, the input device 210 transmits the operation control signal OCS1 instructing to press the third button 215 to the microprocessor 200. Accordingly, the microprocessor 200 or the firmware executed by the microprocessor 200 may control an activation timing and an inactivation timing of each of the control signals CTR1-1, CTR1-2, CTR2-1, and CTR2-2 to perform the second operation mode MODE2 in response to the operation control signal OCS1.

Every time the first button 211 is pressed at time intervals, the microprocessor 200 or the firmware executed by the microprocessor 200 may change operation mode to increase index of the operation mode. Every time the third button 215 is pressed at time intervals, the microprocessor 200 or the firmware executed by the microprocessor 200 may change operation mode to decrease index of the operation mode.

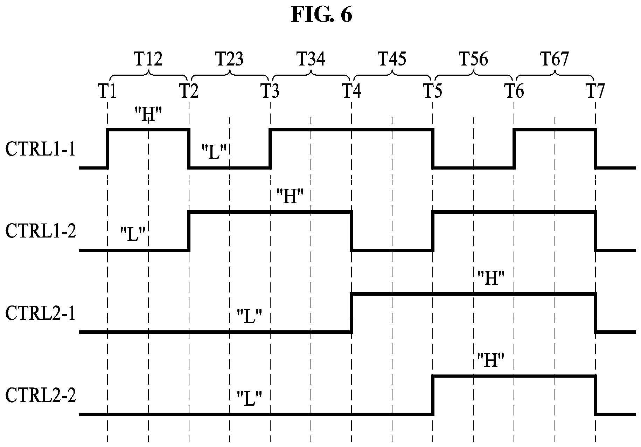

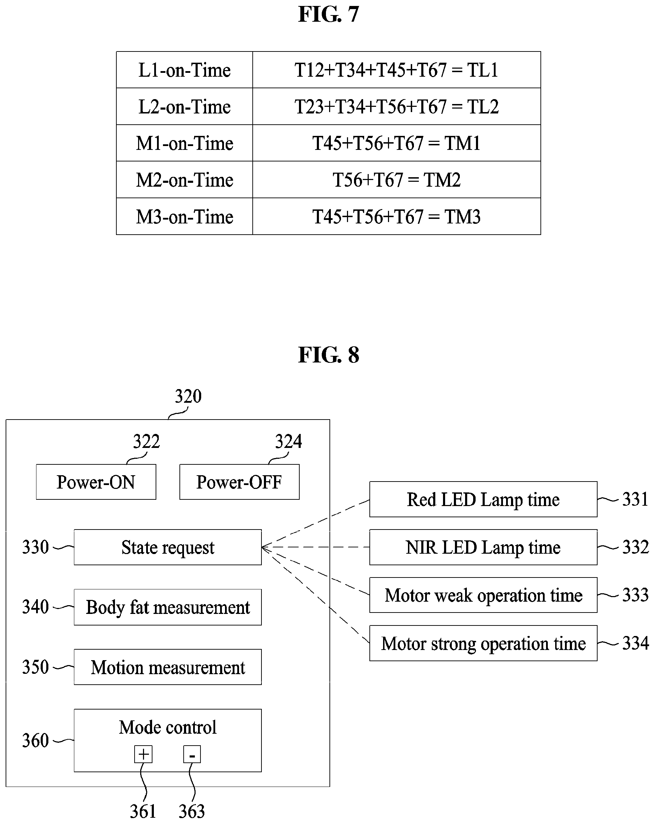

FIG. 6 illustrates a timing diagram of each of control signals of FIG. 4 and FIG. 7 illustrates an example of on-times accumulated by the microprocessor of FIG. 4. Referring to FIG. 1 through 7, a first time section T12 between points in times T1 and T2 is a section in which the first operation mode MODE1 is performed, a second time section T23 between points in times T2 and T3 is a section in which the second operation mode MODE2 is performed, a third time section T34 is a section in which the third operation mode MODE3 is performed, and a fourth time section T45 between points in times T5 and T5 is a section in which the fourth operation mode MODE4 is performed.

Referring to FIG. 7, during an operation of the wearable device 100, the microprocessor 200 or the firmware executed by the microprocessor 200 accumulates and records a time TL1, for example, a first on-time, in which the first LED sources 230 are turned on, accumulates and records a time TL2, for example, a second on-time in which the second LED sources 240 are turned on, and accumulates and records the respective times TM1, TM2, and TM3, for example, a third on-time in which the vibration motors 251, 253, and 255 operate.

FIG. 8 illustrates an example of a graphic user interface (GUI) of an application executed on the mobile device of FIG. 3. The application 320 includes a power-on button 322, a power-off button 324, a state request button 330, a body fat measurement button 340, a motion measurement button 350, and a mode control button 360. Herein, a button may indicate a GUI for generating a signal that controls a specific operation.

If the power-on button 322 is touched or pressed by the user 10, the mobile device 300 may transmit a signal for power-on of the wearable device 100 to the wireless transceiver 270 and the wearable device 100 may start a power-on sequence. According to the power-on sequence or booting, the battery 280 may supply operating voltages to components included in the wearable device 100. If the wearable device 100 is powered on in response to the signal for power-on, the microprocessor 200 or the firmware executed by the microprocessor 200 starts to count a use time or an operation time of the wearable device 100.

If the power-off button 324 is touched or pressed by the user 10, the mobile device 300 may transmit a signal for power-off of the wearable device 100 to the wireless transceiver 270, and the wearable device 100 may start a power-off sequence. According to the power-off sequence, the battery 280 may block operating voltages that are supplied to the components included in the wearable device 100. However, components for the power-on sequence, for example, components capable of waking up the wireless transceiver 270 and the wearable device 100, may be supplied with the operating voltage from the battery 280 at all times.

If the state request button 330 is touched or pressed by the user 10, the mobile device 300 paired with the wearable device 100 may transmit, to the wireless transceiver 270, a state request signal that is generated by the application 320.

In response to the state request signal transmitted from the wireless transceiver 270, the microprocessor 200 may transmit, to the mobile device 300 through the wireless transceiver 270, state information that includes at least one of the accumulatively stored first on-time, the accumulatively stored second on-time, and the accumulatively stored third on-times.

Also, in response to the state request signal transmitted from the wireless transceiver 270, the microprocessor 200 may transmit an operation time of the wearable device 100 to the mobile device 300 as state information through the wireless transceiver 270. The mobile device 300 may provide the state information to the user 10 using the display 310.

Referring to FIG. 3, using the display 310, the application 320 may provide the user 10 with a remaining time from the operation time of the wearable device 100 or a current point in time until the wearable device 100 is automatically powered off based on state information corresponding to the operation time.

The state request button 330 may include a button 331 for verifying an operation time of the first LED sources 230, a button 332 for verifying an operation time of the second LED sources 240, a button 333 for verifying an operation time of the vibration motors 251 and 255, and a button 334 for verifying an operation time of the vibration motor 253.

Here, it is assumed that the vibration motors 251 and 255 operate together when the vibration motor 253 operates. Accordingly, in a motor weak mode, two vibration motors 251 and 255 operate. In a motor strong mode, three vibration motors 251, 253, and 255 operate.

If the body fat measurement button 340 is touched or pressed by the user 10, the mobile device 300 paired with the wearable device 100 may transmit, to the wireless transceiver 270, the body fat measurement signal that is generated by the application 320.

The microprocessor 200 may receive the body fat measurement signal through the wireless transceiver 270, may enable the body fat sensors 291 and 293 in response to the body fat measurement signal, may measure or calculate the body fat, particularly, abdominal body fat of the user 10 based on measurement signals transmitted from the body fat sensors 291 and 293, and may transmit body fat data corresponding to a measurement result to the mobile device 300 through the wireless transceiver 270.

If the motion measurement button 350 is touched or pressed by the user 10, the mobile device 300 paired with the wearable device 100 may transmit, to the wireless transceiver 270, a motion measurement signal that is generated by the application 320.

The microprocessor 200 may receive the motion measurement signal output from the mobile device 300 through the wireless transceiver 270, may enable the motion sensors 295 in response to the motion measurement signal, may measure the motion of the user 10 using measurement signals transmitted from the motion sensors 295, and may transmit motion data corresponding to a measurement result to the mobile device 300 through the wireless transceiver 270.

The mode control button 360 may include a first mode control button 361 and a second mode control button 363. Here, it is assumed that a function of the first mode control button 361 and a function of the first button 211 are the same, and a function of the second mode control button 363 and a function of the third button 215 are the same.

If the mode control button 360 is touched or pressed by the user 10, the mobile device 300 paired with the wearable device 100 may transmit, to the wireless transceiver 270, an operation control signal that is generated by the application 320.

The microprocessor 200 may interpret the operation control signal OCS2 output from the wireless transceiver 270 and may control an activation timing and an inactivation timing of each of the control signals CTR1-1, CTR1-2, CTR2-1, and CTR2-2 based on an interpretation result.

For example, if a current operation mode is the third operation mode MODE3 and, in this instance, the user 10 touches or presses the first mode control button 361, the mobile device 300 transmits, to the wireless transceiver 270, the operation control signal OCS2 instructing to touch or press the first mode control button 361. The microprocessor 200 or the firmware executed by the microprocessor 200 may control activation and inactivation of each of the control signals CTR1-1, CTR1-2, CTR2-1, and CTR2-2 to perform the fourth operation mode MODE4 in response to the operation control signal OCS2 output from the wireless transceiver 270.

However, if the current operation mode is the third operation mode MODE3 and, in this instance, the user 10 touches or presses the second mode control button 363, the mobile device 300 transmits, to the wireless transceiver 270, the operation control signal OCS2 instructing to touch or pressing the second mode control button 363. The microprocessor 200 or the firmware executed by the microprocessor 200 may control activation and inactivation of each of the control signals CTR1-1, CTR1-2, CTR2-1, and CTR2-2 to perform the second operation mode MODE2 in response to the operation control signal OCS2 output from the wireless transceiver 270.

Every time the first mode control button 361 is pressed, the microprocessor 200 or the firmware executed by the microprocessor 200 may change operation mode to increase index of the operation mode. Every time the second mode control button 363 is pressed, the microprocessor 200 or the firmware executed by the microprocessor 200 may change operation mode to decrease index of the operation mode.

Depending on example embodiments, the application 320 may provide the user 10 with a GUI capable of directly selecting one from among the operation modes MODE1 to MODEX of FIG. 5. For example, if the user 10 touches or presses a GUI associated with execution of the third operation mode MODE3 to execute the third operation mode MODE3, the mobile device 300 may transmit, to the wireless transceiver 270, the operation control signal instructing to execute the third operation mode MODE3.

The microprocessor 200 or the firmware executed by the microprocessor 200 may control activation timing and inactivation timing of each of the control signals CTR1-1, CTR1-2, CTR2-1, and CTR2-2 to perform the third operation mode MODE3 by referring to the third operation mode MODE3 among the operation modes stored in the memory device 260, in response to the operation control signal OCS2 output from the wireless transceiver 270.

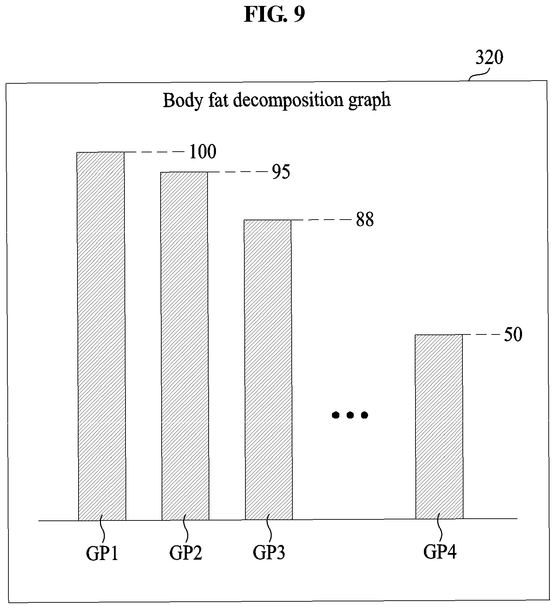

FIG. 9 illustrates an example of a GUI showing a change in a body fat provided from an application executed on the mobile device of FIG. 3. Referring to FIG. 9, the application 300 may process body fat data transmitted from the wearable device 300 to user data and may provide the processed user data to the user 10 through the display 310.

For example, the application 320 may set, as a reference value, for example, "100", body fat data that is initially measured by the user 10, or determined as a default value by the user 10, and may provide the user 10 with body fat data measured subsequently or on another day as a relative value, for example, 95, 88, ..., 50, over the reference value, for example, "100" using the display 310. For example, if the user 10 sets a relative value, for example, "95" as the reference value, the application 320 may recalculate other values, 88, ..., 50, based on the reference value, for example, "95". Here, the application 320 may recalculate other values, for example, 88, ..., 50, based on the reference value, for example, "95" using a proportional equation.

Depending on example embodiments, that is, referring to FIG. 3, the application 320 may calculate a difference, for example, decomposed fat 16.67 g, between a body fat measured on a reference day set by the user 10 and a body fat measured today, and may provide a calculation result to the user 10 through the display 310. Each of bars GP1, GP2, GP3, .., GP4 may represent a body fat measured on a different day or at a different time.

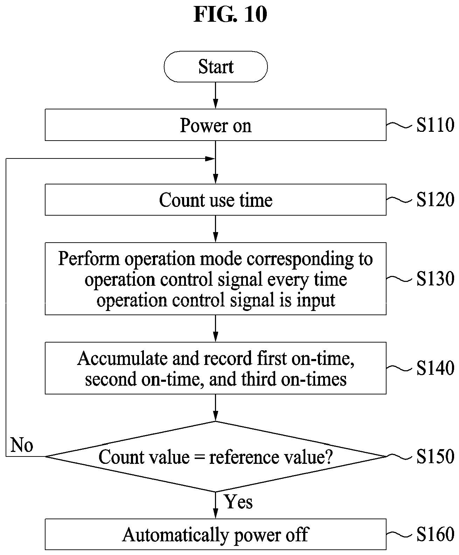

FIG. 10 is a flowchart of an operation of the wearable device of FIG. 4. Referring to FIGS. 1 through 10, in response to the button 213 or 322 being pressed, the wearable device 100 is powered on through a power-on sequence in operation S110.

In operation S120, the microprocessor 200 or firmware executed by the microprocessor 200 counts a use time or an operation time of the wearable device 100 after or immediately after the wearable device 100 is powered on. The microprocessor 200 or the firmware may record a count value in real time.

In operation S130, the microprocessor 200 or the firmware may perform an operation mode corresponding to the operation control signal OCS1 or OCS2 every time the operation control signal OCS1 or OCS2 is received. In operation S130, the microprocessor 200 or the firmware may select one operation mode from among operation modes MODE1 to MODEX stored in the memory device 260 based on the operation control signal OCS1 or OCS2.

In operation S140, the microprocessor 200 or the firmware accumulates and records the operation time, a first on-time, a second on-time, and third on-times. A process of accumulating and recording the first on-time, the second on-time, and the third on-times is described above with reference to FIGS. 6 and 7.

In operation S150, the microprocessor 200 or the firmware may determine whether the count value of the operation time of the wearable device 100 is equal to the reference value, for example, 30 minutes.

If the count value is less than the reference value, the microprocessor 200 or the firmware counts the operation time of the wearable device 100 until the count value reaches the reference value in operation S120. Accordingly, operations 130 and 140 are performed until the count value reaches the reference value.

Once the count value reaches the reference value, the microprocessor 200 or the firmware may automatically power off the wearable device 100 in operation S160.

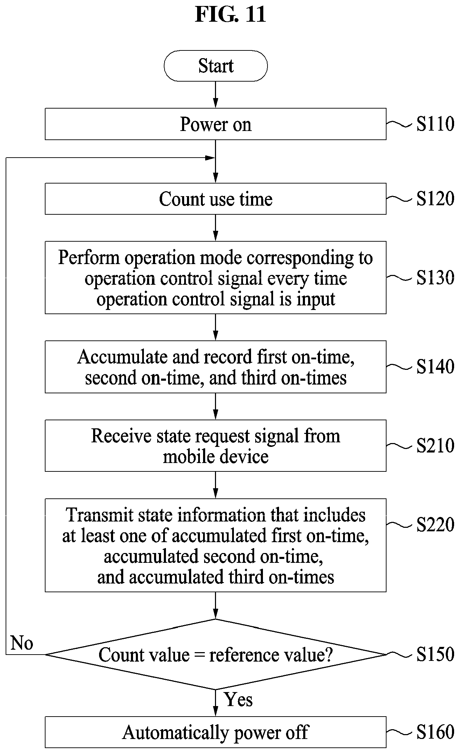

FIG. 11 is a flowchart illustrating a process of acquiring state information from a wearable device using an application executed on the mobile device of FIG. 3.

Referring to FIGS. 10 and 11, operations S110 through S140, S150, and S160 of FIG. 10 are equal to operations of S110 through S140, S150, and S160 of FIG. 11.

If the state request button 330 is touched or pressed by the user 10, the mobile device 300 may transmit, to the wireless transceiver 270, a state request signal that is generated by the application 320. In operation S210, the microprocessor 200 may receive the state request signal transmitted from the wireless transceiver 270. In response to the state request signal, the microprocessor 200 may transmit, to the mobile device 300 through the wireless transceiver 270, state information that includes at least one of the operation time, the accumulated first on-time, the accumulated second on-time, and the accumulated third on-times in operation S220.

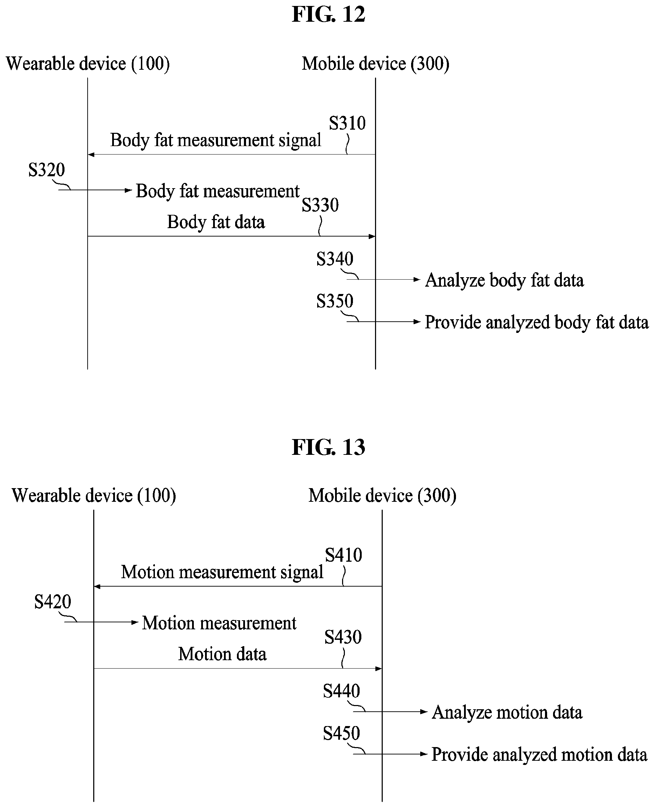

FIG. 12 is a flowchart illustrating a process of controlling a wearable device using an application executed on the mobile device of FIG. 3. Referring to FIGS. 1 through 9 and FIG. 12, if the body fat measurement button 340 is touched or pressed by the user 10, the mobile device 300 may transmit, to the wireless transceiver 270, a body fat measurement signal that is generated by the application 320 in operation S310.

In operation S320, the microprocessor 200 may receive the body fat measurement signal through the wireless transceiver 270, may enable the body fat sensors 291 and 293 in response to the body fat measurement signal, and may measure or calculate a body fat, particularly, abdominal body fat of the user 10 based on measurement signals transmitted from the body fat sensors 291 and 293.

In operation S330, the microprocessor 200 may transmit body fat data corresponding to a measurement result to the mobile device 300 through the wireless transceiver 270. The application 320 of the mobile device 300 may analyze the body fat data in operation S340, and may provide the user 10 with user data corresponding to the analyzed body fat data through the display 310 in operation S350, which is described above with reference to FIGS. 3 and 9.

FIG. 13 is a flowchart illustrating a process of controlling a wearable device using an application executed on the mobile device of FIG. 3. Referring to FIGS. 1 through 9 and FIG. 13, if the motion measurement button 350 is touched or pressed by the user 10, the mobile device 300 may transmit, to the wireless transceiver 270, a motion measurement signal that is generated by the application 320 in operation S410.

In operation S420, the microprocessor 200 may receive the motion measurement signal output from the mobile device 300, may enable the motion sensors 295 in response to the motion measurement signal, and may measure a motion of the user 10 based on measurement signals transmitted from the motion sensors 295. In operation S430, the microprocessor 200 may transmit motion data corresponding to a measurement result to the mobile device 300 through the wireless transceiver 270.

The application 320 of the mobile device 300 may analyze the motion data in operation S440, and may provide the user 10 with user data corresponding to the analyzed motion data through the display 310 in operation S450.

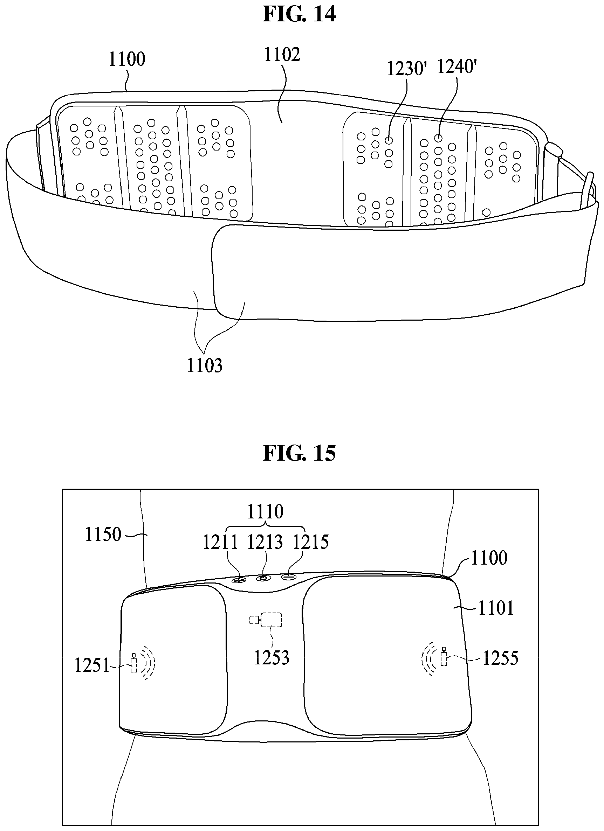

FIG. 14 illustrates a wearable device according to an example embodiment worn around a waist portion of a user, and FIG. 15 illustrates a wearable device according to an example embodiment worn around a waist portion of a user.

Referring to FIGS. 14 and 15, a wearable device 1100 described herein may be a device that is worn around a waist or a waist portion of a user 1150 and is capable of decomposing a body fat, for example, an abdominal fat of the user 1150, and may indicate a diet belt, a wearable light therapy device, or a wearable health care device. Light therapy refers to a technique for applying wavelengths corresponding to a specific color among visible rays to health, beauty, anti-aging, skin care, reduction in obesity, and/or disease protection.

A body of the wearable device 1100 may be formed using an elastic material. A user interface 1110 capable of controlling an operation of the wearable device 1100 may be provided on a front surface 1101 of the wearable device 1100, and the user interface 1110 may include a plurality of buttons 1211, 1213, and 1215.

For example, referring to FIG. 15, the user 1150 wearing the wearable device 1100 around the waist of the user 1150 may adjust power-on and power-off of the wearable device 1100 by manipulating or pressing a second button 1213. The user 1150 may change an operation mode or an operation state by manipulating or pressing each of the buttons 1211 and 1215. Although three buttons 1211, 1213, and 1215 are illustrated in FIGS. 15, 16, and 18 illustrate as an example, they are provided as an example only. A number of buttons associated with an operation of the wearable device 1100 and arrangement locations of the buttons may be variously changed. A belt 1103 is used to couple the wearable device 1100 with the waist or the abdomen of the user 1150.

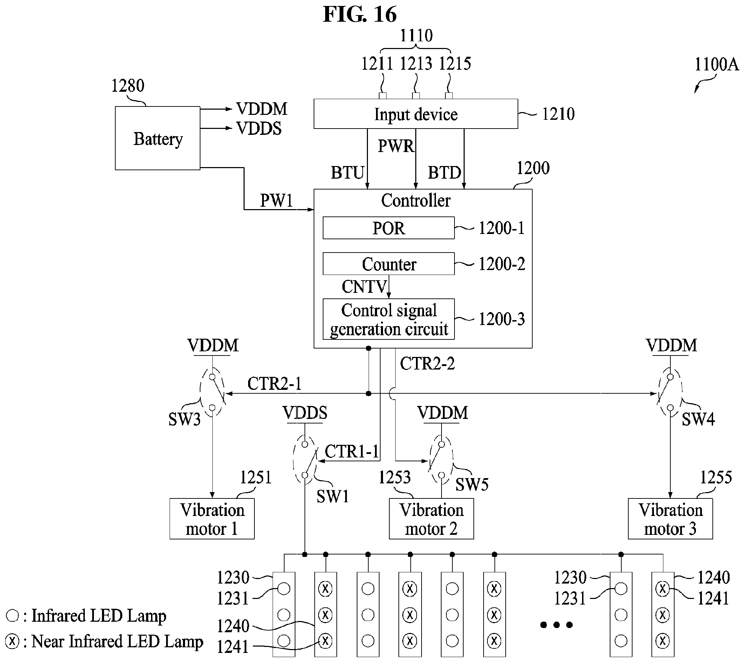

FIG. 16 is a block diagram illustrating an example of an electronic system included in the wearable device of FIGS. 14 and 15. Referring to FIGS. 14 through 16, an electronic system 1100A included in the wearable device 1100 may include a controller 1200, a user interface 1110 including the buttons 1211, 1213, and 1215, an input device 1210, first LED sources 1230, second LED sources 1240, vibration motors 1251, 1253, and 1255, a battery 1280, and switches SW1, SW3, SW4, and SW5.

The controller 1200 may refer to a microprocessor, a processor, or a CPU capable of controlling the overall operation of the electronic system 1100A. An operation of the controller 1200 is described in association with an operation of each of the components 1210, 1230, 1240, 1251, 1253, 1255, and 1280.

The controller 1200 may receive and interpret an operation control signal PWR, BTU, or BTD output from the input device 1210 and may generate a first control signal and a second control signal based on an interpretation result.

The user 1050 that wears the wearable device 1100 around the waist may change power-on, power-off, and/or an operation mode of the wearable device 1100 using each of the buttons 1211, 1213, and 1215 included in the user interface 1110. The operation mode may include a first operation mode in which the LED sources 1230 and 1240 operate, a second operation mode in which the LED sources 1230 and 1240 and the vibration motors 1251 and 1255 operate, and a third operation mode in which the LED sources 1230 and 1240 and the vibration motors 1251, 1253, and 1255 operate.

The input device 1210 may transmit, to the controller 1200, the operation control signal PWR, BTU, or BTD that is generated in response to manipulating or pressing each of the buttons 1211, 1213, and 1215. The input device 1210 may perform a function of generating a signal generated in response to manipulating or pressing each of the buttons 1211, 1213, and 1215 as the operation control signal PWR, BTU, or BTD.

The first LED sources 1230 may be provided to face a rear surface 1102 of the wearable device 1100, that is, the waist or the abdomen of the user 1050 wearing the wearable device 1100, and may output a first light having first wavelengths toward the waist through first holes 1230' formed in the rear surface 1102. The first LED sources 1230 may include first LED lamps 1231. For example, each of the first LED lamps 1231 may be configured as an LED lamp.

The second LED sources 1240 may be provided to face the rear surface 1102 of the wearable device 1100, that is, the waist or the abdomen of the user 1050 wearing the wearable device 1100, and may output a second light having second wavelengths toward the waist through second holes 1240' formed in the rear surface 1102. The second LED sources 1240 may include second LED lamps 1241. For example, each of the second LED lamps 1241 may be configured as an LED lamp configured to generate an NIR ray. A portion of the first wavelengths and a portion of the second wavelengths may be the same.

Although each of the first LED lamps 1231 may be configured as the LED lamp that generates an Red ray and each of the second LED lamps 1241 may be configured as the Led lamp that generates an NIR ray, each of the first LED sources 1230 and each of the second LED sources 1240 may be configured as the same LED lamp depending on example embodiments.

An Red ray or an NIR ray may penetrate into the skin around the waist portion of the user 1050 and be transferred to fat cells of panniculus.

Once a light, for example, the Red ray and/or NIR ray output from each LED lamp 1231 and/or 1241 is emitted toward the waist portion or the abdominal portion of the user 1050, the mitochondria may generate energy for decomposing a fat. Such a chemical variation may promote the secretion of lipase enzymes that decompose triglyceride into fat acid and glycerol. A lipid material decomposed into small pieces may be discharged through pores formed on the surface of the fat cells and the discharged lipid material may naturally decline or be combusted by a movement of the user 1050 while circulating in a lymphatic system of the body.

If the wearable device 1100 applies a vibration to the waist portion using at least one of the vibration motors 1251, 1253, and 1255 while emitting a light toward the waist portion or the abdominal portion of the user 1050 using each LED lamp 1231 and/or 1241, the motility of fat cells may increase and the lipid material decomposed into small pieces may move smoothly in the body. Accordingly, the vibration may decrease the abdominal fat in replacement of aerobic exercise.

All of the first LED sources 1230 and the second LED sources 1240 may be turned on in response to an activated first source control signal CTR1-1. Here, the first control signal indicates a first source control signal CTR1-1.

For example, if the first source control signal CTR1-1 is activated, the first switch SW1 simultaneously supplies a source operating voltage VDDS to the first LED sources 1230 and the second LED sources 1240. Thus, the first LED sources 1230 and the second LED sources 1240 may emit a light using the source operating voltage VDDS.

The controller 1200 may control an activation timing and an inactivation timing of the first source control signal CTR1-1 based on the first operation control signal PWR output from the input device 1210.

The vibration motors 1251 and 1255 that perform functionality of micro vibrators may operate, for example, vibrate in response to the activated first motor control signal CTR2-1. The vibration motor 1253 may operate, for example, vibrate in response to an activated second motor control signal CTR2-2. The second control signal refers to all of the motor control signals CTR2-1 and CTR2-2. Accordingly, at least one of the vibration motors 1251 and 1255 may be enabled or disabled in response to the second control signal (CTR2-1 and CTR2-2).

For example, if the first motor control signal CTR2-1 is activated, third switch SW3 may supply a motor operating voltage VDDM to the first vibration motor 1251 and the first vibration motor 1251 may be enabled or vibrate. Also, if the first motor control signal CTR2-1 is activated, the fourth switch SW4 may supply a motor operating voltage VDDM to the third vibration motor 1255 and the third vibration motor 1255 may be enabled or vibrate. If the second motor control signal CTR2-2 is activated, the fifth switch SW5 may supply the motor operating voltage VDDM to the second vibration motor 1253 and the second vibration motor 1253 may be enabled or vibrate.

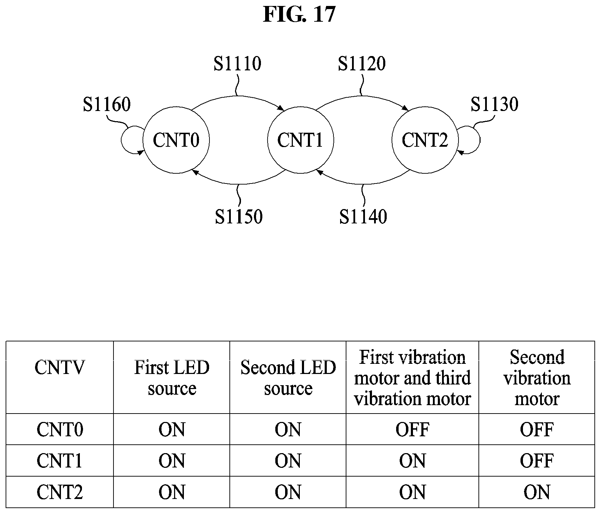

Although FIG. 17 illustrates an example in which two vibration motors 1251 and 1255 operate in response to the activated first motor control signal CTR2-1 and a single vibration motor 1253 operates in response to the activated second motor control signal CTR2-2, a number of vibration motors and an operation order of the vibration motors provided in the wearable device 1100 may vary based on example embodiments. FIG. 15 illustrates the vibration motors 1251, 1253, and 1255 that are provided in the wearable device 1100.

The controller 1200 may control an activation timing and an inactivation timing of each of the motor control signals CTR2-1 and CTR2-2 in response to the operation control signal BTU or BTD output from the input device 1210.

The controller 1200 may execute firmware or software. The firmware or the software may perform a function of counting an operation time of the wearable device 110 when an operation of the wearable device 1100 starts, a function of comparing a count value corresponding to a count result and a reference value, and a function of automatically turning off power VDDM, VDDS, and PW1 supplied to the wearable device 1100 when the count value is equal to the reference value. For example, the firmware or the software may control a power supply function of the battery 1280.

The controller 1200 may receive the operation control signal PWR, BTU, or BTD output from the input device 1210, may change a count value CNTV, as shown in FIG. 17, based on the operation control signal PWR, BTU, or BTD, and may generate the first control signal CTR1-1 and the second control signal (CTR2-1 and CTR2-2) based on the changed count value CNTV. That is, the controller 1200 may control an activation timing and an inactivation timing of each of the control signals CTR1-1, CTR2-1, and CTR2-2 based on the count value CNTV that is determined based on the operation control signal PWR, BTU, or BTD.

The battery 1280 may output operating voltages PW1, VDDM, and VDDS. The operating voltage PW1 is supplied to the controller 1200, the motor operating voltage VDDM is supplied to the vibration motors 1251, 1253, and 1255 through the respective corresponding switches SW3, SW4, and SW5, and the source operating voltage VDDS is supplied to each of the LED source 1230 and 1240 through the first switch SW1.

FIG. 17 is provided to describe an operation of the controller of FIG. 16. Referring to FIGS. 16 and 17, the controller 1200 may include a counter 1200-2. The counter 1200-2 may be configured as hardware and may be configured as software executed by the controller 1200. The controller 1200 may include a power-on reset (POR) circuit 1200-1. The POR circuit 1200-1 may control the operating voltage PW1 to be supplied to the controller 1200 in response to the first operation control signal PWR.

If the second button 1213 is pressed, the input device 1210 may output the first operation control signal BTU instructing power-on to the controller 1200. A POR circuit 1200-1 of the controller 1200 may be powered on in response to the first operation control signal PWR instructing power-on and the counter 1200-2 may generate a count value CNTV having a first value CNTO.

In response to the count value CNTV having the first value CNTO, a control signal generation circuit 1200-3 may generate the first source control signal CTR1-1 that is activated at a high level, the first motor control signal CTR2-1 having the low level, and the second motor control signal CTR2-2 having the low level. Accordingly, the first LED sources 1230) and the second LED sources 1240 may emit the light using the source operating voltage VDDS.

If the count value CNTV is the first value CNTO and, in this instance, the first button 1211 is pressed, the input device 1210 outputs, to the controller 1200, the second operation control signal BTU instructing to change the operation mode. In operation S1110, the counter 1200-2 generates a count value CNTV having a second value CNT1 in response to the second operation control signal BTU. Here, the second value CNT1 is greater than the first value CNT0.

In response to the count value CNTV having the second value CNT1, the control signal generation circuit 1200-3 generates the first source control signal CTR1-1 having the high level, the first motor control signal CTR2-1 that is activated at the high level, and the second motor control signal CTR2-2 having the low level. Accordingly, the first LED sources 1230 and the second LED sources 1240 emit the light using the source operating voltage VDDS and the vibration motors 1251 and 1255 operate or vibrate using the motor operating voltage VDDM.

If the count value CNTV is the second value CNT1 and, in this instance, the first button 1211 is pressed again, the input device 1210 outputs, to the controller 1200, the second operation control signal BTU instructing to change the operation mode. In operation S1120, the counter 1200-2 generates a count value CNTV having a third value CNT2 in response to the second operation control signal BTU. Here, the third value CNT2 is greater than the second value CNT1.

In response to the count value CNTV having the third value CNT2, the control signal generation circuit 1200-3 generates the first source control signal CTR1-1 having the high level, the first motor control signal CTR2-1 having the high level, and the second motor control signal CTR2-2 that is activated at the high level. Accordingly, the first LED sources 1230 and the second LED sources 1240 emit the light using the source operating voltage VDDS, and all of the vibration motors 1251, 1253, and 1255 operate or vibrate using the motor operating voltage VDDM.

If the count value CNTV is the first value CNT0, the wearable device 1100 performs the first operation mode. If the count value CNTV is the second value CNT1, the wearable device 1100 performs the second operation mode. If the count value CNTV is the third value CNT2, the wearable device 1100 performs the third operation mode.

However, if the count value CNTV is the second value CNT1 and, in this instance, the third button 1215 is pressed, the input device 1210 outputs, to the controller 1200, the third operation control signal BTD instructing to change the operation mode. In operation S1150, the counter 1200-2 generates a count value CNTV that decreases from the second value CNT1 from the first value CNT0 in response to the third operation control signal BTD.

In response to the count value CNTV having the first value CNT0, the control signal generation circuit 1200-3 generates the first source control signal CTR1-1 having the high level, the first motor control signal CTR2-1 that is activated at the low level, and the second motor control signal CTR2-2 having the low level. Accordingly, the first LED sources 1230 and the second LED sources 1240 emit the light using the source operating voltage VDDS. Alternatively, the vibration motors 1251 and 1255 are turned off.

If the count value CNTV is the third value CNT2 and, in this instance, the third button 1215 is pressed, the input device 1210 outputs, to the controller 1200, the third operation control signal BTD instructing to change the operation mode. In operation S1140, the counter 1200-2 generates a count value CNTV that decreases from the third value CNT2 to the second value CNT1 in response to the third operation control signal BTD.

In response to the count value CNTV having the second value CNT1, the control signal generation circuit 1200-3 generates the first source control signal CTR1-1 having the high level, the first motor control signal CTR2-1 having the high level, and the second motor control signal CTR2-2 that is activated at the low level. Accordingly, the first LED sources 1230 and the second LED sources 1240 emit the light using the source operating voltage VDDS. Only the third vibration motor 1253 is turned off.

If the count value CNTV is the third value CNT2 and, in this instance, the first button 1211 is pressed, the input device 1210 outputs, to the controller 1200, the second operation control signal BTU instructing to change the operation mode. However, in operation S1130, the counter 1200-2 generates a count value CNTV that maintains the third value CNT2 instead of increasing the third value CNT2 in response to the second operation control signal BTU.

If the count value CNTV is the first value CNT0 and, in this instance, the third button 1215 is pressed, the input device 1210 outputs, to the controller 1200, the third operation control signal BTD instructing to change the operation mode. However, in operation S1160, the counter 1200-2 generates a count value CNTV that maintains the first value CNT0, instead of decreasing the first value CNT0 in response to the third operation control signal BTD.

As described above with reference to FIGS. 16 and 17, the controller 1200 generates the first control signal CTR1-1 in response to the first operation control signal PWR, increases the count value or the current count value by counting a number of inputs of the second operation control signal BTU every time the second operation control signal BTU is input, decreases the count value or the current count value by counting a number of inputs of the third operation control signal BTD every time the third operation control signal BTD is input, and generates the second control signal (CTR2-1 and CTR2-2) corresponding to the increased count value or the decreased count value.

A first switch circuit SW1 controls on or off of the first LED sources 1230 and the second LED sources 1240 in response to the first control signal CTR1-1. Here, the first switch circuit SW1 includes the first switch SW1.

Second switch circuits SW3, SW4, and SW5 may adjust a number of vibration motors to operate among the vibration motors 1251, 1253, and 1255 based on the second control signal (CTR2-1 and CTR2-2). The second switch circuits SW3, SW4, and SW5 include the third switch SW3, the fourth switch SW4, and the fifth switch SW5.

If the second operation control signal BTU is input after the count value CNTV reaches a first count value, for example, CNT2, the controller 1200 maintains the first count value, for example, CNT2. If the third operation control signal BTD is input after the count value CNTV reaches a second count value, for example, CNT0, the controller 1200 maintains the second count value, for example, CNT0. Here, the first count value, for example, CNT2, is greater than the second count value, for example, CNT0.

The controller 1200 generates the activated first control signal CTR1-1 in response to the first operation control signal PWR that is input at a first point in time, and the first switch circuit SW1 supplies the first operating voltage VDDS, that is, the source operating voltage VDDS among the operating voltages VDDM, VDDS, and PW1, to at least one of the first LED sources 1230 and the second LED sources 1240 in response to the activated first control signal CTR1-1.

At a second point in time at which a predetermined time, for example, 30 minutes, from the first point in time, has elapsed, the controller 1200 may automatically inactivate the activated first control signal CTR1-1 to block the first operating voltage VDDS supplied to at least one of the first LED source 1230 and the second LED source 1240. That is, the controller 1200 may automatically power off the wearable device 1100. The first point in time indicates a point in time at which the operating voltage PW1 is supplied to the wearable device 1100 in a power-off state in response to the second button 1213 being pressed.

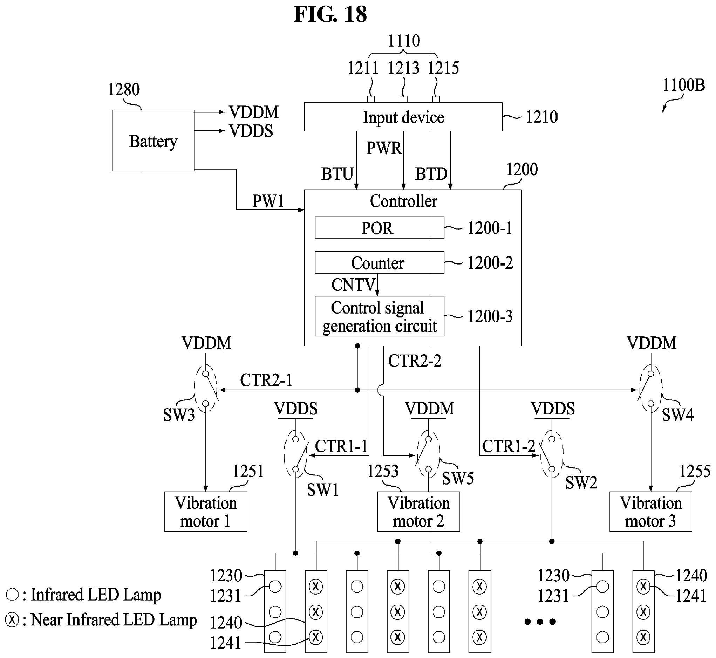

FIG. 18 is a block diagram illustrating another example embodiment of the electronic system included in the wearable device of FIGS. 14 and 15, and FIG. 19 is provided to describe an operation of a controller of FIG. 18.

Referring to FIG. 18, an electronic system 1100B included in the wearable device 1100 may include a controller 1200, a user interface 1110 including buttons 1211, 1213, and 1215, an input device 1210, first LED sources 1230, second LED sources 1240, vibration motors 1251, 1253, and 1255, the battery 1280, and switches SW1, SW2, SW3, SW4, and SW5.

The first LED sources 1230 may be turned on in response to an activated first source control signal CTR1-1 and the second LED sources 1240 may be turned on in response to an activated second source control signal CTR1-2. Here, the first control signal refers to all of the source control signals CTR1-1 and CTR1-2.

For example, if the first source control signal CTR1-1 is activated, the first switch SW1 may supply a source operating voltage VDDS to the first LED sources 1230 and the first LED sources 1230 may emit a light using the source operating voltage VDDS. For example, if the second source control signal CTR1-2 is activated, the second switch SW2 may supply the source operating voltage VDDS to the second LED sources 1240 and the second LED sources 1240 may emit the light using the source operating voltage VDDS.

If the second button 1213 is pressed, the input device 1210 outputs a first operation control signal BTU instructing power-on to the controller 1200. A POR circuit 1200-1 of the controller 1200 is powered on in response to the first operation control signal PWR instructing power-on and a counter 1200-2 generates a count value CNTV having a first value CNT0.

In response to the count value CNTV having the first value CNT0, a control signal generation circuit 1200-3 generates the first source control signal CTR1-1 that is activated at a high level, the first source control signal CTR1-2 having a low level, the first motor control signal CTR2-1 having the low level, and the second motor control signal CTR2-2 having the low level. Since the first switch SW1 supplies the source operating voltage VDDS to the first LED sources 1230 in response to the first source control signal CTR1-1 that is activated at the high level, the first LED sources 1230 may emit the light using the source operating voltage VDDS.

If the count value CNTV is the first value CNT0 and, in this instance, the first button 1211 is pressed, the input device 1210 outputs, to the controller 1200, the second operation control signal BTU instructing to change the operation mode. In operation S1210, the counter 1200-2 generates a count value CNTV having the second value CNT1 in response to the second operation control signal BTU.

In response to the count value CNTV having the second value CNT1, the control signal generation circuit 1200-3 generates the first source control signal CTR1-1 having the high level, the second source control signal CTR1-2 that is activated at the high level, the first motor control signal CTR2-1 having the low level, and the second motor control signal CTR2-2 having the low level. Accordingly, only the first LED sources 1230 and the second LED sources 1240 emit the light using the source operating voltage VDDS.

If the count value CNTV is the second value CNT1 and, in this instance, the first button 1211 is pressed again, the input device 1210 outputs, to the controller 1200, the second operation control signal BTU instructing to change the operation mode. In operation S1220, the counter 1200-2 generates a count value CNTV having a third value CNT2.

In response to the count value CNTV having the third value CNT2, the control signal generation circuit 1200-3 generates the first source control signal CTR1-1 having the high level, the second source control signal CTR1-2 having the high level, the first motor control signal CTR2-1 that is activated at the high level, and the second motor control signal CTR2-2 having the low level. Accordingly, the first LED sources 1230 and the second LED sources 1240 emit the light using the source operating voltage VDDS. The vibration motors 1251 and 1255 operate or vibrate using the motor operating voltage VDDM.

If the count value CNTV is the third value CNT2 and, in this instance, the first button 1211 is pressed again, the input device 1210 outputs, to the controller 1200, the second operation control signal BTU instructing to change the operation mode. In operation S1230, the counter 1200-2 generates a count value CNTV having a fourth value CNT3 in response to the second operation control signal BTU. Here, the fourth value CNT3 is greater than the third value CNT2.

In response to the count value CNTV having the fourth value CNT3, the control signal generation circuit 1200-3 generates the first source control signal CTR1-1 having the high level, the second source control signal CTR1-2 having the high level, the first motor control signal CTR2-1 having the high level, and the second motor control signal CTR2-2 that is activated at the high level. Accordingly, the first LED sources 1230 and the second LED sources 1240 emit the light using the source operating voltage VDDS. The vibration motors 1251 and 1255 operate or vibrate using the motor operating voltage VDDM.

The operation mode of the wearable device 1100 may include a first operation mode in which the LED sources 1230 operate, a second operation mode in which the LED sources 1230 and 1240 operate, a third operation mode in which the LED sources 1230 and 1240, and the vibration motors 1251 and 1255 operate, and a fourth operation mode in which the LED sources 1230 and 1240 and the vibration motors 1251, 1253, and 1255 operate.

If the count value CNTV is the fourth value CNT3 and, in this instance, the first button 1211 is pressed, the input device 1210 outputs, to the controller 1200, the second operation control signal BTU instructing to change the operation mode. However, in operation S1240, the counter 1200-2 generates a count value CNTV that maintains the fourth value CNT3, instead of increasing the fourth value CNT3 in response to the second operation control signal BTU.

However, if the count value CNTV is the fourth value CNT3 and, in this instance, the third button 1215 is pressed, the input device 1210 outputs, to the controller 1200, the third operation control signal BTD instructing to change the operation mode. In operation S1250, the counter 1200-2 generates a count value CNTV having the third value CNT2 in response to the third operation control signal BTD.

In response to the count value CNTV having the third value CNT2, the control signal generation circuit 1200-3 generates the first source control signal CTR1-1 having the high level, the second source control signal CTR1-2 having the high level, the first motor control signal CTR2-1 having the high level, and the second motor control signal CTR2-2 that is activated at the low level. Accordingly, the first LED sources 1230 and the second LED sources 1240 emit the light using the source operating voltage VDDS and only the second vibration motor 1253 among the vibration motors 1251, 1253, and 1255 is turned off.

If the count value CNTV is the third value CNT2 and, in this instance, the third button 1215 is pressed, the input device 1210 outputs, to the controller 1200, the third operation control signal BTD instructing to change the operation mode. In operation S1260, the counter 1200-2 generates a count value CNTV having the second value CNT1 in response to the third operation control signal BTD.

In response to the count value CNTV having the second value CNT1, the control signal generation circuit 1200-3 generates the first source control signal CTR1-1 having the high level, the second source control signal CTR1-2 having the high level, the first motor control signal CTR2-1 that is activated at the low level, and the second motor control signal CTR2-2 that is activated at the low level. Accordingly, the first LED sources 1230 and the second LED sources 1240 emit the light using the source operating voltage VDDS and all of the vibration motors 1251, 1253, and 1255 are turned off.

If the count value CNTV is the second value CNT1 and, in this instance, the third button 1215 is pressed, the input device 1210 outputs, to the controller 1200, the third operation control signal BTD instructing to change the operation mode. In operation S1270, the counter 1200-2 generates a count value CNTV having the first value CNT0 in response to the third operation control signal BTD.

In response to the count value CNTV having the first value CNT0, the control signal generation circuit 1200-3 generates the first source control signal CTR1-1 having the high level, the second source control signal CTR1-2 that is activated at the low level, the first motor control signal CTR2-1 having the low level, and the second motor control signal CTR2-2 having the low level. Accordingly, the first LED sources 1230 emit the light using the source operating voltage VDDS and the second LED sources 1240 are turned off.

If the count value CNTV is the first value CNT0 and, in this instance, the third button 1215 is pressed, the input device 1210 outputs, to the controller 1200, the third operation control signal BTD instructing to change the operation mode. In operation S1280, the counter 1200-2 generates the count value CNTV having the first value CNT0 in response to the third operation control signal BTD.

FIG. 16 illustrates an example embodiment in which, if the second button 1213 is pressed, the first LED sources 1230 and the second LED sources 1240 operate at default, for example, are turned on. However, if the second button 1213 is pressed, the electronic system 1100A may maintain only a standby state. If the first button 1211 is initially pressed, the electronic system 1100A may be changed to operate, for example, turn on the first LED sources 1230 and the second LED sources 1240.

In this case, if the first button 1211 is initially pressed, the counter 1200-2 may generate the count value CNTV having the first value CNT0. If the count value CNTV is the first value CNT0 and, in this instance, the first button 1211 is pressed, the counter 1200-2 may generate the count value CNTV having the second value CNT1. If the count value CNTV is the second value CNT1 and, in this instance, the first button 1211 is pressed, the counter 1200-2 may generate the count value CNTV having the third value CNT2.

FIG. 18 illustrates an example embodiment in which, if the second button 1213 is pressed, the first LED sources 1230 operate at default, for example, are turned on. However, if the second button 1213 is pressed, the electronic system 1100B may maintain only a standby state. If the first button 1211 is initially pressed, the electronic system 1100B may be changed to operate, for example, turn on the first LED sources 1230. In this case, if the first button 1211 is initially pressed, the counter 1200-2 may generate the count value CNTV having the first value CNT0.

If the count value CNTV is the first value CNT0 and, in this instance, the first button 1211 is pressed, the counter 1200-2 may generate the count value CNTV having the second value CNT1. If the count value CNTV is the second value CNT1 and, in this instance, the first button 1211 is pressed, the counter 1200-2 may generate the count value CNTV having the third value CNT2. If the count value CNTV is the third value CNT2 and, in this instance, the first button 1211 is pressed, the counter 1200-2 may generate the count value CNTV having the fourth value CNT3.

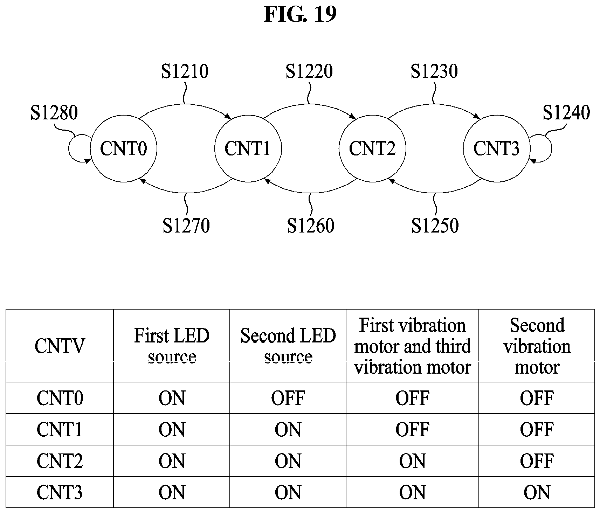

As described above with reference to FIGS. 18 and 19, the controller 1200 may be enabled in response to the first operation control signal PWR, may increase a count value by counting a number of inputs of the second operation control signal BTU every time the second operation control signal BTU is input, may decrease the count value by counting a number of inputs of the third operation control signal BTD every time the third operation control signal BTD is input, and may generate the first control signal (CTR1-1 and CTR1-2) and the second control signal (CTR2-1 and CTR2-2) corresponding to the increased count value or the decreased count value.

A first switch circuit including SW1 and SW2 may adjust a number of LED sources to operate among the first LED sources 1230 and the second LED sources 1240 in response to the first control signal (CTR1-1 and CTR1-2). The first switch circuit (SW1 and SW2) includes the first switch SW1 and the second switch SW2.

A second switch circuit including (SW3 to SW5) may adjust a number of vibration motors to operate among the vibration motors 1251, 1253, and 1255 based on the second control signals CTR2-1 and CTR2-2. The second switch circuit (SW3-SW5) includes the third switch SW3 to the fifth switch SW5.

If the second operation control signal BTU is input after the count value CNTV reaches a first count value, for example, CNT3, the controller 1200 maintains the first count value, for example, CNT3. If the third operation control signal BTD is input after the count value CNTV reaches a second count value, for example, CNT0, the controller 1200 maintains the second count value, for example, CNT0. Here, the first count value, for example, CNT3, is greater than the second count value, for example, CNT0.

Although the present disclosure is described with reference to the accompanying drawings, it is provided as an example only. It will be apparent to those of ordinary skills in the art that various modifications and equivalent other example embodiments may be made from the description. Accordingly, the true technical scope of the disclosure is to be defined by the claims.

### INDUSTRIAL APPLICABILITY

Example embodiments relate to a wearable device that may reduce a body fat.

## Claims

1. A wearable device comprising:
a first light emitting diode (LED) source configured to emit a first light having first wavelengths;
a second LED source configured to emit a second light having second wavelengths;
vibration motors;
a microprocessor configured to interpret an operation control signal and to generate a first control signal and a second control signal based on a result of the interpreting; and
a battery configured to supply operating voltages to the first LED source, the second LED source, the vibration motors, and the microprocessor,
wherein at least one of the first LED source and the second LED source is turned on in response to the first control signal,
at least one of the vibration motors is turned on in response to the second control signal, and
the microprocessor is configured to accumulate and record a first on-time of the first LED source, a second on-time of the second LED source, and third on-times of the vibration motors.

2. The wearable device of claim 1, further comprising:
a memory device configured to store information associated with operation modes,
wherein the microprocessor is configured to select one of the operation modes stored in the memory device using the operation control signal, and to generate the first control signal and the second control signal based on the selected operation mode.

3. The wearable device of claim 1, wherein the microprocessor is configured to execute firmware for accumulating and recording an operation time of the wearable device, the first on-time, the second on-time, and the third on-times.

4. The wearable device of claim 1, further comprising:
a wireless transceiver configured to connect to the microprocessor,
wherein the microprocessor is configured to transmit state information including at least one of an operation time of the wearable device, the accumulated first on-time, the accumulated second on-time, and the accumulated third on-times to a mobile device through the wireless transceiver in response to a state request signal transmitted from a wireless transceiver, and
the state request signal is transmitted from the mobile device.

5. The wearable device of claim 1, further comprising:
an input device configured to connect to the microprocessor; and
a wireless transceiver configured to wirelessly communicate with a mobile device paired with the wearable device, and to connect to the microprocessor,
wherein the operation control signal is output from one of the input device and the wireless transceiver.

6. The wearable device of claim 5, further comprising:
body fat sensors configured to measure a body fat of a user that wears the wearable device around the waist,
wherein the microprocessor is configured to receive a body fat measurement signal output from the mobile device through the wireless transceiver, to enable the body fat sensors in response to the body fat measurement signal, to measure the body fat of the user using measurement signals transmitted from the body fat sensors, and to transmit body fat data corresponding to a measurement result to the mobile device through the wireless transceiver.

7. The wearable device of claim 6, further comprising:
a motion sensor configured to measure a motion of the user that wears the wearable device around the waist,
wherein the microprocessor is configured to receive a motion measurement signal output from the mobile device through the wireless transceiver, to enable the motion sensor in response to the motion measurement signal, to measure the motion of the user using a measurement signal transmitted from the motion sensor, and to transmit motion data corresponding to a measurement result to the mobile device through the wireless transceiver.

8. The wearable device of claim 5, wherein the input device includes a first user interface and a second user interface,
a number of LED sources turned on in response to the first control signal between the first LED source and the second LED source when the operation control signal is a first operation control signal input through the first user interface is greater than a number of LED sources turned on in response to the first control signal between the first LED source and the second LED source when the operation control signal is a second operation control signal input through the second user interface, and
a number of vibration motors turned on in response to the second control signal among the vibration motors when the operation control signal is a third operation control signal input through the first user interface is greater than a number of vibration motors turned on in response to the second control signal among the vibration motors when the operation control signal is a fourth operation control signal input through the second user interface.

9. The wearable device of claim 1, wherein the microprocessor is configured to detect power-on of the wearable device, to count an operation time of the wearable device based on a detection result, and to automatically power off the wearable device when a count value reaches a reference value.

10. The wearable device of claim 1, wherein the first LED source includes Red LED lamps, and
the second LED source includes near infrared (NIR) LED lamps

11. A wearable device in a structure to be wearable around the waist of a user to measure a body fat of the user, the wearable device comprising:
a first light emitting diode (LED) source configured to emit a first light having first wavelengths toward the waist;
a second LED source configured to emit a second light having second wavelengths toward the waist;
vibration motors configured to provide a vibration to the waist;
an input device configured to provide a user interface to the user;
a wireless transceiver configured to wirelessly communicate with a mobile device;
a body fat sensors configured to measure the body fat; and
a microprocessor configured to interpret an operation control signal input through one of the input device and the wireless transceiver and to generate a first control signal and a second control signal based on a result of the interpreting,
wherein at least one of the first LED source and the second LED source is turned on in response to the first control signal,
at least one of the vibration motors is turned on in response to the second control signal, and
the microprocessor is configured to accumulate and store a first on-time of the first LED source, a second on-time of the second LED source, and third on-times of the vibration motors.

12. The wearable device of claim 11, further comprising:
a memory device configured to store information associated with operation modes,
wherein the microprocessor is configured to select one of the operation modes stored in the memory device using the operation control signal, and to generate the first control signal and the second control signal based on the selected operation mode.

13. The wearable device of claim 12, wherein the microprocessor is configured to transmit state information including at least one of an operation time of the wearable device, the accumulated first on-time, the accumulated second on-time, and the accumulated third on-times to a mobile device through the wireless transceiver in response to a state request signal transmitted from a wireless transceiver, and
the state request signal is transmitted from the mobile device.

14. The wearable device of claim 13, wherein the microprocessor is configured to receive a body fat measurement signal output from the mobile device through the wireless transceiver, to enable the body fat sensors in response to the body fat measurement signal, to measure the body fat of the user using measurement signals transmitted from the body fat sensors, and to transmit body fat data corresponding to a measurement result to the mobile device through the wireless transceiver.

15. The wearable device of claim 13, further comprising:
a motion sensor configured to measure a motion of the user,
wherein the microprocessor is configured to receive a motion measurement signal output from the mobile device through the wireless transceiver, to enable the motion sensor in response to the motion measurement signal, to measure the motion of the user using a measurement signal transmitted from the motion sensor, and to transmit motion data corresponding to a measurement result to the mobile device through the wireless transceiver.

16. A method of controlling an operation of a wearable device using the wearable device in a structure to be wearable around the waist of a user to measure a body fat of the user and a mobile device capable of wirelessly communicating with the wearable device, the method comprising:
receiving, by a microprocessor included in the wearable device, an operation control signal input through one of an input device included in the wearable device and a wireless transceiver included in the wearable device;
selecting, by the microprocessor, one of operation modes of the wearable device from a memory device configured to store information associated with the operation modes using the operation control signal, and to generate a first control signal and a second control signal based on the selected operation mode;
turning on at least one of a first light emitting diode (LED) source and a second LED source included in the wearable device in response to the first control signal, and turning on at least one of vibration motors included in the wearable device in response to the second control signal;
accumulating and recording, by the microprocessor, a first on-time of the first LED source, a second on-time of the second LED source, and third on-times of the vibration motors;
receiving, by the microprocessor, a state request signal transmitted from a wireless communication device through the wireless transceiver; and
transmitting, by the microprocessor, at least one of an operation time of the wearable device, the accumulated first on-time, the accumulated second on-time, and the accumulated third on-times to the mobile device through the wireless transceiver in response to the state request signal.

17. The method of claim 16, further comprising:
receiving, by the microprocessor, a body fat measurement signal output from the mobile device through the wireless transceiver;
enabling, by the microprocessor, body fat sensors included in the wearable device in response to the body fat measurement signal; and
measuring, by the microprocessor, the body fat of the user using measurement signals transmitted from the body fat sensors, and transmitting body fat data corresponding to a measurement result to the mobile device through the wireless transceiver.

18. The method of claim 16, further comprising:
receiving, by the microprocessor, a motion measurement signal transmitted from the mobile device through the wireless transceiver;
enabling, by the microprocessor, a motion sensor included in the wearable device in response to the motion measurement signal; and
measuring, by the microprocessor, the motion of the user using a measurement signal transmitted from the motion sensor, and transmitting motion data corresponding to a measurement result to the mobile device through the wireless transceiver.
